# FASCICULE DE BREVET EUROPEEN

(11) **EP 1 753 781 B1**
(45) Date de publication et mention de la délivrance du brevet: **26.09.2012**
(21) Numéro de dépôt: 05770968.5
(22) Date de dépôt: 10.05.2005
(51) Int. Cl.: C07K 14/685, C07K 7/06, C07K 5/04, A61K 47/48

(54) **PEPTIDES OU CONJUGUES PEPTIDIQUES DERIVES DE LA MSH ET LEUR UTILISATION DANS LE TRAITEMENT COSMETIQUE DE LA CANITIE**
MSH-PEPTIDE ODER PEPTIDISCHE KONJUGATDERIVATE UND DEREN VERWENDUNG FÜR DIE KOSMETISCHE BEKÄMPFUNG VON GRAUEM HAAR (CANITIES)
PEPTIDES OR PEPTIDIC CONJUGATE DERIVATIVES OF MSH AND THE USE THEREOF FOR COSMETICALLY FIGHTING AGAINST CANITIES

(30) Priorité: 11.05.2004 FR 0405069
(43) Date de publication de la demande: 21.02.2007
(73) Titulaire: INSTITUT EUROPEEN DE BIOLOGIE CELLULAIRE, 31520 Ramonville St Agne (FR); Centre National de la Recherche Scientifique, 75016 Paris (FR); Université de Montpellier I, 34006 Montpellier Cedex 1 (FR); Université Montpellier II, 34095 Montpellier Cedex 05 (FR)
(72) Inventeur: HOCQUAUX, Michel, F-75012 Paris (FR); PINEL, Anne-Marie, F-31400 Toulouse (FR); MARTINEZ, Jean, F-34720 Caux (FR); SUBRA, Gilles, F-34990 Juvignac (FR)
(74) Mandataire: Faivre Petit, Frédérique
(86) Numéro de dépôt international: PCT/FR2005/001166
(87) Numéro de publication internationale: WO 2005/116068

(56) Documents cités:
- WO-A-01/98362
- WO-A-02/085925
- WO-A-03/064458
- WO-A-03/095474
- US-A- 5 714 576
- US-A- 5 786 332
- US-A- 5 830 994
- HASKELL-LUEVANO C ET AL: "Truncation studies of alpha-melanotropin peptides identify tripeptide analogues exhibiting prolonged agonist bioactivity" PEPTIDES, ELMSFORD, US, vol. 17, no. 6, 1996, pages 995-1002, XP002970858 ISSN: 0196-9781
- HOLDER JERRY RYAN ET AL: "Structure-activity relationships of the melanocortin tetrapeptide Ac-His-D-Phe-Arg-Trp-NH2 at the mouse melanocortin receptors. 4. Modifications at the Trp position." JOURNAL OF MEDICINAL CHEMISTRY, vol. 45, no. 26, 19 décembre 2002 (2002-12-19), pages 5736-5744, XP002302531 ISSN: 0022-2623
- HOLDER J R ET AL: "Characterization of aliphatic, cyclic, and aromatic N-terminally capped His-D-Phe-Arg-Trp-NH2 tetrapeptides at the melanocortin receptors" EUROPEAN JOURNAL OF PHARMACOLOGY, AMSTERDAM, NL, vol. 462, 21 février 2003 (2003-02-21), pages 41-52, XP002263329 ISSN: 0014-2999
- HOLDER J R ET AL: "STRUCTURE-ACTIVITY RELATIONSHIPS OF THE MELANOCORTIN TETRAPEPTIDE AC-HIS-DPHE-ARG-TRP-NH2 AT THE MOUSE MELANOCROTIN RECEPTORS. 1. MODIFICATIONS AT THE HIS POSITION" JOURNAL OF MEDICINAL CHEMISTRY, AMERICAN CHEMICAL SOCIETY. WASHINGTON, US, vol. 45, no. 13, 20 juin 2002 (2002-06-20), pages 2801-2810, XP001183565 ISSN: 0022-2623
- TAKAHAMA MOTOHIDE: "alpha-MSH discovered in primary root of sesame seeds, and trial on remelanization of gray hairs by their extract: immunohistochemical study" JOURNAL OF DERMATOLOGICAL SCIENCE, vol. 34, no. 2, avril 2004 (2004-04), page 148, XP002302532 & 29TH ANNUAL MEETING OF THE JAPANESE SOCIETY FOR INVESTIGATIVE DERMATOLOGY; KYOTO, JAPAN; APRIL 14-16, 2004 ISSN: 0923-1811
- NIJENHUIS WOUTER A J ET AL: "Discovery and in vivo evaluation of new melanocortin-4 receptor-selective peptides." PEPTIDES (NEW YORK), vol. 24, no. 2, février 2003 (2003-02), pages 271-280, XP002345048 ISSN: 0196-9781

## Description

L'invention concerne de nouveaux peptides ou conjugués peptidiques dérivés de la MSH et leur utilisation dans le traitement cosmétique de la canitie ou la lutte contre le blanchiment des cheveux.

La couleur des cheveux est déterminée par la concentration dans les kératinocytes de la mélanine produite par les mélanocytes. La pigmentation du cheveu nécessite la présence de mélanocytes au niveau du bulbe du follicule pileux. Les mélanocytes sont des cellules spécialisées qui synthétisent la mélanine. Deux groupes de pigments mélaniques sont formés : les eumélanines et les phéomélanines. Ces pigments sont transmis aux kératinocytes qui forment la tige pilaire. La synthèse de la mélanine ou mélanogénèse fait intervenir la tyrosine qui sous l'action d'une tyrosinase se transforme en mélanine. La tyrosinase est l'enzyme essentielle intervenant dans cette cascade de réaction.
Avec l'apparition des rides, le blanchiment des cheveux est l'un des premiers signes du vieillissement. Les causes d'apparition des cheveux blancs peuvent être multiples et leurs interactions très mal connues : facteurs génétiques, processus de vieillissement, environnement, mode de vie. De nombreuses hypothèses sont émises pour expliquer le processus de vieillissement. L'apparition de cheveux blancs est associée à :
1. Une diminution du nombre de mélanocytes dans les bulbes pileux.
2. Une diminution de la quantité de mélanine dans la tige pilaire.
Les éléments biochimiques de la coloration sont toujours présents mais inactifs : la fabrication et le transport des mélanines sont progressivement stoppés. 3. Un défaut de transfert de mélanine à partir des mélanosomes dans les kératinocytes. La production de la mélanine au niveau des follicules pileux s'effectue juste au-dessus de la papille dermique. C'est à ce niveau que s'effectue le transfert de mélanine vers les kératinocytes qui donnent naissance à la tige pilaire.

La présente invention porte sur des peptides et conjugués peptidiques dérivés de la MSH.
La MSH ou "Melanocyte Stimulating Hormone" est l'hormone de la pigmentation. La MSH est un régulateur très important de la mélanogénèse. Exp. Dermatol. 1998/07/43-150*.* La MSH induit la prolifération mélanocytaire et la synthèse de mélanine.

La présente invention repose sur la recherche de structures peptidiques, activatrices de la mélanogénèse au niveau des mélanocytes du bulbe pileux.
Ainsi la demanderesse a découvert de façon surprenante que de nouveaux peptides et conjugués peptidiques contenant au plus 6 aminoacides et comprenant la séquence - DPhe - Arg - avait une action très importante d'activation de la mélanogénèse au niveau des mélanocytes du bulbe pileux.

La demande de brevet EP 669 938 décrit des séquences peptidiques comportant au moins la séquence His-Phe-Arg. Toutefois, la phenylalanine peut se trouver sous sa forme D ou L. De plus il est indiqué qu'avantageusement Phe représente homoPhe ou p-fluoroPhe. En outre, dans les exemples seul le D.homoPhe est utilisé et il est par ailleurs précisé que la présence de ce D.homoPhe est très importante pour la stimulation de la mélanogénèse et l'activation de la tyrosinase. Enfin, ce document ne suggère pas que ces peptides aient une action d'activation de la mélanogénèse au niveau des mélanocytes du bulbe pileux et puissent traiter la canitie ou lutter contre le blanchiment des cheveux.
La demande de brevet WO 03/064458 décrit des conjugués peptidiques de formule R-V-Ala-His-X-Y-Trp-NH₂, dans lesquels X peut représenter la phenylalanine sous forme D ou L, Y l'arginine et A un groupe acétyle.
Ainsi le peptide Ac-Nle-Ala-His-DPhe-Arg-Trp-NH₂ est décrit.
Toutefois, ces conjugués peptidiques commencent obligatoirement par la séquence Nle-Ala-His et se terminent obligatoirement par la séquence Trp-NH₂. De plus X et Y peuvent représenter d'autres acides aminés que la phénylalanine ou l'arginine. En outre, les peptides ou conjugués peptidiques selon la présente invention ont une activité supérieure sur la mélanogénèse par rapport au peptide Ac-Nle-Ala-His-DPhe-Arg- Trp-NH₂.
Le brevet US 5 714 576 décrit des fragments linéaires analogues de la MSH pouvant, entre autres, stimuler la mélanogénèse. Toutefois, les tests fournis n'ont été réalisés que sur les grenouilles. En outre ces fragments contiennent au minimum 7 aminoacides au lieu de 6 au maximum comme dans la présente invention. De plus ce document ne suggère pas que des fragments plus petits puissent avoir une activité sur la mélanogénèse. En outre, lorsque les fragments décrits dans ce document contiennent la séquence Hist-DPhe-Arg-Trp, un aminoacide est toujours présent après le Trp et ces séquences ne contiennent jamais d'aminoacide final Trp NH₂, ce qui n'est pas le cas dans la présente invention.

La présente invention concerne un conjugué peptidique selon la revendication 1.

Les acides aminés dans le conjugué peptidique selon l'invention peuvent avoir une configuration D, L ou DL si cela n'est pas spécifié autrement.
Ainsi, les conjugués peptidiques peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques font partie de l'invention.
Les conjugués peptidiques sont des dérivés de faible poids moléculaire qui sont obtenus sous forme d'amides. De plus, les conjugués peptidiques peuvent être couplés avec du zinc, sous forme de sel pour former des complexes.

Dans le cadre de la présente invention, on entend par :
- Lys, la lysine,
- Arg, l'arginine,
- Trp, le tryptophane,
- Nap, la naphtylalanine,
- Tpi, l'acide tétrahydronorhaman - 3 carboxylique,
- Tic, l'acide tétrahydroisoquinoline - 3 carboxylique,
- Ala, l'alanine,
- Phe, la phénylalanine,
- Nle, la norleucine,
- His, l'histidine et
- Orn, l'ornithine.

Il est également précisé que les conjugués peptidiques faisant l'objet de la présente invention, sont obtenus sous la forme terminale NH₂ (autrement dit présentant une fonction amide).

Les conjugués peptidiques de l'invention sont choisis parmi :
2- Ac-Nle-Ala-Arg-DPhe-Arg-Trp-NH₂
8- Prop-Nle-Ala-His-DPhe-Arg-Trp-NH₂
9- But-Nle-Ala-His-DPhe-Arg-Trp-NH₂
10- Palm-Nle-Ala-His-DPhe-Arg- Trp-NH₂
11- Lip-Nle-Ala-His-DPhe-Arg-Trp-NH₂
14- Ac-Nle-Ala-His-DPhe-Arg-Nap-NH₂
20- Ac-Nle-Trp-His-DPhe-Arg-Trp-NH₂
22- Palm-His-DPhe-Arg-Trp-NH₂
24- Palm-His-DPhe-Arg-Ala-NH₂
25- Pbu-His-DPhe-Arg-Trp-NH₂
26- Lip-His-DPhe-Arg-Trp-NH₂

Les conjugués peptidiques objet de la présente invention, peuvent être obtenus soit avantageusement par synthèse chimique classique, soit par synthèse enzymatique, selon des procédés quelconques connus de l'homme du métier.

Les peptides ou leurs conjugués peptidiques peuvent être administrés pour leur utilisation cosmétique par voie topique. Ils peuvent aussi être utilisés dans des compléments alimentaires, autrement dit dans le domaine nutraceutique par voie orale.
Les conjugués peptidiques selon l'invention sont préférentiellement administrés, par voie topique.

Selon un autre aspect, la présente invention a également pour objet une composition cosmétique, dermatologique ou pharmaceutique ou encore un complément alimentaire comprenant un peptide ou un conjugué peptidique selon la présente invention.

La composition cosmétique ou dermatologique peut avantageusement être appliquée sur l'ensemble du cuir chevelu.

La composition cosmétique ou dermatologique peut par exemple se présenter sous forme de lotions, shampoings traitants, sprays, après-shampoings, crèmes, pommades, solutions, émulsions, gels, laits, masques, sérums.

Dans la composition cosmétique topique, le conjugué peptidique ou le peptide selon l'invention peut être présent à une concentration comprise entre 10⁻⁸ M et 10⁻³ M, de préférence comprise entre 10⁻⁷ M et 10⁻⁵ M.

Enfin, un autre objet de la présente invention concerne un procédé de traitement cosmétique pour lutter contre le blanchiment des cheveux et/ou traiter la canitie comprenant l'application sur le cuir chevelu d'une composition comprenant un peptide ou un conjugué peptidique de l'invention ou encore comprenant l'administration par voie orale d'un complément alimentaire contenant une peptide ou un conjugué peptidique de l'invention.

Les compositions cosmétiques selon la présente invention, destinées à l'application topique sur le cuir chevelu, peuvent en outre comprendre un filtre UVB permettant la photoprotection du cuir chevelu. Ainsi, parmi les filtres UVB adaptés on peut citer, sous leur nom INCI :
- L'acide p-aminobenzoïque ou PABA et ses esters :
   * EthylhexyldimethylPABA
   * PEG-25PABA
- Les cinnamates :
   * Ethylhexyl Methoxycinnamate
   * Isoamyl p-Methoxycinnamate
   * Octocrylene
- les Salicylates :
   * Homosalate
   * Ethylhexyl Salicylate
- les benzimidazoles :
   * acide de Phenylbenzimidazole sulfonique
- les dérivés Benzylidène camphres
   * 4-Methylbenzylidene Camphre
   * Benzilidene Camphre
   * Camphre Benzalkonium Methosulfate
   * Polyacrylamidomethyl Benzylidene Camphre
- les triazines :
   * Ethylhexyl Triazone
   * Diethylhexyl Butamido Triazone.

Les peptides de l'invention ont fait l'objet d'essais pharmacologiques permettant de montrer leur activité dans le traitement de la canitie. Les exemples suivants sont donnés à titre indicatif non limitatif.

### Exemple 1 : Activité de différents peptides dans un test de stimulation de la mélanogénèse

### Test : Etude de la quantification du second messager : l'AMPc

L'α-MSH possède au niveau de la peau le récepteur 1 à la mélanocortine MClr. La liaison d'un agoniste avec ce récepteur active une molécule effectrice, l'adénylate cyclase, qui produit l'AMPc. En quantifiant l'AMPc, on évalue l'affinité d'un ligand pour son récepteur.

Protocole : les cellules utilisées qui expriment le MClr sont des mélanocytes humains. Les peptides sont testés aux concentrations de 10⁻⁶ à 10⁻¹¹ M. La molécule de référence l'α-MSH est testée aux mêmes concentrations.

La EC₅₀ correspond à la concentration de ligand donnant 50% de la stimulation maximale. Les EC₅₀ reportées dans le tableau 1 ci-après sont exprimées en nM, correspondant à la moyenne de 3 expérimentations.

| **Conjugué peptidique** | **EC50** |
|---|---|
| alpha- MSH | 8,50 |
| peptide art antérieur | 1,8 |
| Conjugué n°2 | 0,6 |
| Conjugué n°8 | 1 |
| Conjugué n°10 | 1,6 |

Les peptides de l'invention présentent une activité supérieure à l'alpha-MSH

### Exemple 2 : Lotion comprenant le conjugué peptidique 11

| | En g |
|---|---|
| Conjugué peptidique 11 | 20.10⁻⁶ |
| Ethanol à 95° | 60 |
| Propylène glycol | 10 |
| Eau/Conservateurs | qsp 100 |

### Exemple 3 : Lotion comprenant le conjugué peptidique 24

| | En g |
|---|---|
| Conjugué peptidique 24 | 20.10⁻⁶ |
| Eau | 81 |
| Keltrol® T (gomme de xanthane commercialisée par la société KECCO) | 0,5 |
| Techpolymer MB-4C (polymethyl Méthacrylate) | 1 |
| Commercialise par la société Sekisui Sepigel® 305 | 0,5 |
| (polyacrilamide/C₁₃-C₁₄ Isoparafine/ Laureth-7 commercialisée par la société SEPPIC) | |
| Huile de silicone | 2 |
| Butylène glycol | 5 |

Deux applications par jour (matin et soir) pendant 3 mois de la lotion de l'exemple 2 sur une femme n'ayant que des cheveux blancs, ont permis de visualiser l'apparition de cheveux repigmentés noirs.

## Revendications

1. Conjugué peptidique choisi parmi :
Ac-Nle-Ala-Arg-DPhe-Arg-Trp-NH₂
Prop-Nle-Ala-His-DPhe-Arg-Trp-NH₂
But-Nle-Ala-His-DPhe-Arg-Trp-NH₂
Palm-Nle-Ala-His-DPhe-Arg-Trp-NH₂
Lip-Nle-Ala-His-DPhe-Arg-Trp-NH₂
Ac-Nle-Ala-His-DPhe-Arg-Nap-NH₂
Ac-Nle-Trp-His-DPhe-Arg-Trp-NH₂
Palm-His-DPhe-Arg-Trp-NH₂
Palm-His-DPhe-Arg-Ala-NH₂
Pbu-His-DPhe-Arg-Trp-NH₂
Lip-His-DPhe-Arg-Trp-NH₂

2. Utilisation d'un conjugué peptidique selon la revendication 1 pour le traitement cosmétique de la canitie et/ou la lutte contre le blanchiment des cheveux.

3. Procédé de traitement cosmétique pour lutter contre le blanchiment des cheveux et/ou traiter la canitie **caractérisée en ce qu'**il comprend l'application sur le cuir chevelu d'une composition cosmétique contenant un conjugué peptidique selon la revendication 1.

4. Procédé de traitement cosmétique pour lutter contre le blanchiment des cheveux et/ou traiter la canitie **caractérisée en ce qu'**il comprend l'administration par voie orale d'un complément alimentaire contenant un conjugué peptidique selon la revendication 1.

## Claims

1. Peptidic conjugate chosen from among:
Ac-Nle-Ala-Arg-DPhe-Arg-Trp-NH₂
Prop-Nle-Ala-His-DPhe-Arg-Trp-NH₂
But-Nle-Ala-His-DPhe-Arg-Trp-NH₂
Palm-Nle-Ala-His-DPhe-Arg-Trp-NH₂
Lip-Nle-Ala-His-DPhe-Arg-Trp-NH₂
Ac-Nle-Ala-His-DPhe-Arg-Nap-NH₂
Ac-Nle-Trp-His-DPhe-Arg-Trp-NH₂
Palm-His-DPhe-Arg-Trp-NH₂
Palm-His-DPhe-Arg-Ala-NH₂
Pbu-His-DPhe-Arg-Trp-NH₂
Lip-His-DPhe-Arg-Trp-NH₂

2. Use of a peptidic conjugate of formula (I) for the cosmetic treatment of canities and/or to help prevent whitening hair.

3. A cosmetic treatment process to help prevent whitening hair and/or to treat canities wherein it consists in the application to the scalp of a cosmetic composition containing a peptide according to claim 1.

4. A cosmetic treatment process to help prevent whitening hair and/or to treat canities wherein it consists in administration by oral route of a food supplement containing a peptide according to claim 1.

## Patentansprüche

1. Peptid-Konjugat, ausgewählt aus:
Ac-Nle-Ala-Arg-DPhe-Arg-Trp-NH₂
Prop-Nle-Ala-His-DPhe-Arg-Trp-NH₂
But-Nle-Ala-His-DPhe-Arg-Trp-NH₂
Palm-Nle-Ala-His-DPhe-Arg-Trp-NH₂
Lip-Nle-Ala-His-DPhe-Arg-Trp-NH₂
Ac-Nle-Ala-His-DPhe-Arg-Nap-NH₂
Ac-Nle-Trp-His-DPhe-Arg-Trp-NH₂
Palm-His-DPhe-Arg-Trp-NH₂
Palm-His-DPhe-Arg-Ala-NH₂
Pbu-His-DPhe-Arg-Trp-NH₂
Lip-His-DPhe-Arg-Trp-NH₂

2. Verwendung eines Peptid-Konjugats nach Anspruch 1 zur kosmetischen Behandlung von Canities und/oder zur Bekämpfung von Haarergrauung.

3. Kosmetisches Behandlungsverfahren zur Bekämpfung von Haarergrauung und/oder zur Behandlung von Canities, **dadurch gekennzeichnet, dass** es die Anwendung einer kosmetischen Zusammensetzung, die ein Peptid-Konjugat nach Anspruch 1 enthält, auf der Kopfhaut umfasst.

4. Kosmetisches Behandlungsverfahren zur Bekämpfung von Haarergrauung und/oder zur Behandlung von Canities, **dadurch gekennzeichnet, dass** es die orale Verabreichung eines Nahrungsergänzungsmittels, das ein Peptid-Konjugat nach Anspruch 1 enthält, umfasst.
